# FASCICULE DE BREVET EUROPEEN

(11) **EP 0 634 164 B1**
(45) Date de publication et mention de la délivrance du brevet: **11.11.1998**
(21) Numéro de dépôt: 94401612.0
(22) Date de dépôt: 12.07.1994
(51) Int. Cl.: A61K 7/13

(54) **Composition de teinture d'oxydation des fibres kératiniques comprenant un para-aminophénol, le 2-méthyl 5-aminophénol et une paraphénylènediamine et/ou une bis-phénylalkylènediamine**
Oxydationsfärbemittel für keratinische Fasern enthaltend ein Para-Aminophenol, 2-Methyl-5-Aminophenol und ein Paraphenylendiamin und/oder ein Bis-Phenylalkylendiamin
Oxidative dying composition for keratinous fibers containing a para-aminophenol, 2-methyl-5-aminophenil and a paraphenylenediamine and/or a bis-phenylalkylenediamine

(30) Priorité: 13.07.1993 FR 9308616
(43) Date de publication de la demande: 18.01.1995
(73) Titulaire: L'OREAL, 75008 Paris (FR)
(72) Inventeur: Cotteret, Jean, F-78480 Verneuil-sur-Seine (FR); Audousset, Marie Pascale, F-92300 Levallois-Perret (FR); Lagrange, Alain, F-77700 Coupvray (FR); Vandenbosche, Jean-Jacques, F-93279 Sevran (FR)
(74) Mandataire: Bulle, Françoise

(56) Documents cités:
- EP-A- 0 182 187
- EP-A- 0 241 716
- EP-A- 0 459 901
- WO-A-93/00066
- FR-A- 2 315 255
- FR-A- 2 421 869

## Description

La présente invention est relative à une composition de teinture des fibres kératiniques, et en particulier des fibres kératiniques humaines, comprenant, en association, au moins un para-aminophénol, le 2-méthyl 5-aminophénol et au moins une paraphénylènediamine et/ou une bis-phénylalkylènediamine, et au procédé de teinture utilisant une telle composition et mettant en oeuvre la révélation par un agent oxydant.

Il est connu de teindre les fibres kératiniques et en particulier les cheveux humains, avec des compositions tinctoriales contenant des précurseurs de colorants d'oxydation, en particulier des ortho- ou paraphénylènediamines, des ortho- ou para-aminophénols, généralement appelés "bases d'oxydation", et des coupleurs encore appelés modificateurs de coloration, plus particulièrement des méta-phénylènediamines aromatiques, des méta-aminophénols et des méta-diphénols, qui permettent de modifier et d'enrichir de reflets les colorations "de fond" obtenues par les produits de condensation des bases d'oxydation.

On recherche, dans le domaine de la teinture capillaire d'oxydation, des précurseurs de colorants d'oxydation ainsi que des coupleurs qui permettent de conférer aux cheveux une coloration ayant une résistance satisfaisante à la lumière, aux lavages, aux intempéries, à la transpiration et aux différents traitements que peuvent subir les cheveux, et d'obtenir un large éventail de nuances de coloration.

Le 3-méthyl para-aminophénol et le 2-hydroxymethyl para-aminophénol ainsi que leur utilisation dans des compositions tinctoriales pour fibres kératiniques en association avec le 2-méthyl 5-aminophénol à titre de coupleur et la paraphénylènediamine ou le 2,5-diaminotoluène, sont connus et décrits respectivement dans le brevet US-4.883.656 et dans la demande de brevet EP-A-0182187.

Cependant, de telles associations ne procurent pas, après application sur les fibres kératiniques, une coloration suffisamment résistante.

La demanderesse vient de découvrir, ce qui fait l'objet de l'invention, que l'utilisation du 3-méthyl para-aminophénol, du 2-méthyl para-aminophénol et/ou du 2-hydroxyméthyl para-aminophénol à titre de précurseurs de colorants d'oxydation, en association avec le 2-méthyl 5-aminophénol à titre de coupleur et au moins une paraphénylènediamine et/ou une bis-phénylalkylènediamine de formule (I) ou (II) définie ci-après à titre de précurseur de colorant d'oxydation, permet d'obtenir, en présence d'un agent oxydant, en milieu acide ou alcalin, après application sur les fibres kératiniques et en particulier les cheveux humains, des colorations aux nuances rouges ou cuivrées et présentant une bonne résistance à la lumière, aux lavages, aux intempéries, à la transpiration et aux différents traitements que peuvent subir les cheveux.

La présente invention a donc pour objet une composition de teinture des fibres kératiniques, en particulier des fibres kératiniques humaines telles que les cheveux, comprenant, dans un milieu approprié pour la teinture :
- au moins un précurseur de colorant d'oxydation choisi parmi le 3-méthyl para-aminophénol, le 2-méthyl para-aminophénol et le 2-hydroxyméthyl para-aminophénol, et leurs sels d'addition avec un acide;
- au moins, à titre de coupleur, le 2-méthyl 5-aminophénol, ou l'un de ses sels d'addition avec un acide; et
- au moins un précurseur de colorant d'oxydation choisi parmi les paraphénylènediamines et les bis-phénylalkylènediamines de formules (I) et (II) ci-après, ainsi que leurs sels d'addition avec un acide.

L'invention a également pour objet un agent de teinture à plusieurs composants, dont le premier composant contient les précurseurs de colorants d'oxydation et le coupleur définis ci-dessus et le deuxième composant un agent oxydant.

Un autre objet de l'invention porte sur la composition prête à l'emploi, contenant les différents agents utilisés pour la teinture des fibres kératiniques définis ci-dessus et un agent oxydant, en milieu alcalin ou acide.

L'invention vise également un procédé de teinture des fibres kératiniques, et en particulier des fibres kératiniques humaines telles que les cheveux, consistant à appliquer sur ces fibres :
- au moins un précurseur de colorant d'oxydation choisi parmi le 3-méthyl para-aminophénol, le 2-méthyl para-aminophénol et le 2-hydroxyméthyl para-aminophénol, et leurs sels d'addition avec un acide;
- au moins, à titre de coupleur, le 2-méthyl 5-aminophénol, ou l'un de ses sels d'addition avec un acide;
- au moins une paraphénylènediamine de formule (I) définie ci-après et/ou au moins une bis-phénylalkylènediamine de formule (II) définie ci-après, ou l'un de leurs sels d'addition avec un acide à titre de précurseur de colorant d'oxydation;
la couleur étant révélée par un agent oxydant en milieu acide ou alcalin.

Selon l'invention et parmi les précurseurs de type para-aminophénol ci-dessus, le 3-méthyl p-aminophénol est préféré.

Les paraphénylènediamines utilisables selon l'invention, répondent à la formule (I) ci-après : 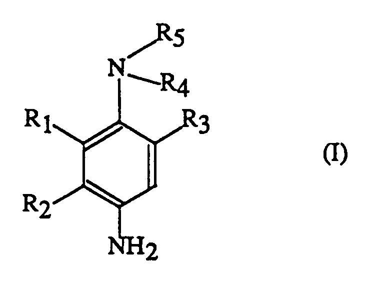 dans laquelle :
R₁, R₂, R₃, identiques ou différents, représentent un atome d'hydrogène ou d'halogène, un radical alkyle, un radical alcoxy, un radical carboxy, sulfo ou hydroxyalkyle en C₁-C₄;
R₄ et R₅, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino; ou bien R₄ et R₅ forment conjointement avec l'atome d'azote auquel ils sont liés, un hétérocycle pipéridino ou morpholino, sous réserve que R₁ ou R₃ représente un atome d'hydrogène lorsque R₄ et R₅ ne représentent pas un atome d'hydrogène, et sous réserve que lorsque R₁, R₂ et R₃ désignent un atome d'hydrogène, l'un des radicaux R₄ et R₅ soit différent d'hydrogène, et lorsque R₄ et R₅ désignent un atome d'hydrogène et que l'un des radicaux R₁, R₂ ou R₃ représente un radical méthyle, au moins l'un des deux autres soit différent d'hydrogène. Ces radicaux alkyle ou alcoxy ont de préférence 1 à 4 atomes de carbone et désignent notamment les radicaux méthyle, éthyle, propyle, méthoxy et éthoxy.

Parmi les composés de formule (I), on peut plus particulièrement citer la méthoxyparaphénylènediamine, la chloroparaphénylène diamine, la 2,3-diméthylparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylène diamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl, carbamylméthyl)aniline, la 3-méthyl 4-amino N,N-(éthyl, carbamylméthyl)aniline, la 4-amino N,N-(éthyl,β-pipéridinoéthyl) aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl) aniline, la N-[(4'-amino)phényl]morpholine, la N-[(4'-amino)phényl]pipéridine, la 2-β-hydroxyéthylparaphénylènediamine, la fluoroparaphénylènediamine, la carboxyparaphénylènediarnine, la sulfoparaphénylènediamine, la 2-isopropylparaphénylènediamine, la 2-n-propylparaphénylènediamine, la N-(β-hydroxypropyl)paraphénylènediamine, la 2-hydroxyméthylparaphénylènediamine, la N,N-diméthyl 3-méthylparaphénylènediamine, la N,N-(éthyl,β-hydroxyéthyl)paraphénylènediamine, la N-(dihydroxypropyl)paraphénylènediamine, la N-(4'-aminophényl)paraphénylènediamine, la N-phénylparaphénylènediamine.

Ces paraphénylènediamines peuvent être utilisées soit sous forme de base libre, soit sous forme de sels, tels que chlorhydrate, bromhydrate ou sulfate.

Parmi les composés de formule (I), on préfère :
- la 2,6-diméthylparaphénylènediamine,
- la 2-β-hydroxyéthylparaphénylènediamine,
- la 2-hydroxyméthylparaphénylènediamine,
- la 2-n-propylparaphénylènediamine,
- la 2-isopropylparaphénylènediamine,
- la N-(β-hydroxypropyl)paraphénylènediamine,
- la N,N-di-(β-hydroxyéthyl)paraphénylènediamine,
- la 4-amino N-(β-méthoxyéthyl)aniline,
et leurs sels.

Les bases dites doubles sont des bis-phénylalkylènediamines, répondant à la formule : 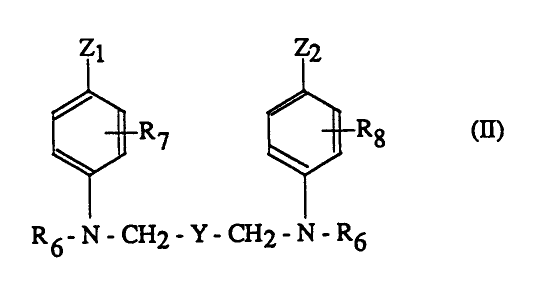 dans laquelle :
Z₁ et Z₂, identiques ou différents, représentent des groupements hydroxyle ou NHR₉, où R₉ désigne un atome d'hydrogène ou un radical alkyle inférieur;
R₇ et R₈, identiques ou différents, représentent soit des atomes d'hydrogène, soit des atomes d'halogène, soit encore des groupements alkyle ;
R₆ représente un atome d'hydrogène, un groupe alkyle, hydroxyalkyle ou aminoalkyle, dont le reste amino peut être substitué;
Y représente un radical pris dans le groupe constitué par les radicaux suivants : -(CH₂)ₙ-, -(CH₂)ₘ-O-(CH₂)ₘ-, -(CH₂)ₘ-CHOH-(CH₂)ₘ-, 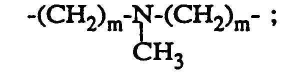 n étant un nombre entier compris entre 0 et 8 et m un nombre entier compris entre 0 et 4, ces bases pouvant se présenter également sous forme de leurs sels d'addition avec des acides.

Les radicaux alkyle ou alcoxy ci-dessus indiqués désignent de préférence un groupement ayant 1 à 4 atomes de carbone et notamment méthyle, éthyle, propyle, méthoxy et éthoxy.

Parmi les composés de formule (II), on peut citer le N,N'-bis-(β-hydroxyéthyl)N,N'-bis-(4'-aminophényl) 1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl) N,N'-bis-(4-aminophényl)tétraméthylènediamine, la N,N'-bis-(4-méthylaminophényl)tétraméthylènediamine, la N,N'-bis-(éthyl)N,N'-bis-(4'-amino 3'-méthylphényl)éthylènediamine, et leurs sels.

Le N,N'-bis-(β-hydroxyéthyl)N,N'-bis-(4'-aminophényl) 1,3-diamino 2-propanol est particulièrement préféré.

Selon le procédé conforme à l'invention, on applique sur les fibres kératiniques humaines, au moins une composition (A) contenant dans un milieu approprié pour la teinture :
- au moins un précurseur de colorant d'oxydation choisi parmi le 3-méthyl para-aminophénol, le 2-méthyl para-aminophénol, le 2-hydroxyméthyl para-aminophénol, et leurs sels;
- au moins, à titre de coupleur, le 2-méthyl 5-aminophénol, ou l'un de ses sels;
- au moins une paraphénylènediamine de formule (I) et/ou une bisphénylalkylènediamine de formule (II), ou leurs sels, à titre de précurseur de colorant d'oxydation,
la couleur étant révélée en milieu acide ou alcalin, à l'aide d'un agent oxydant ajouté juste au moment de l'emploi à la composition (A) ou présent dans une composition (B) appliquée simultanément ou séquentiellement de façon séparée.

L'invention a également pour objet des dispositifs de teinture ou "kits", à plusieurs compartiments, permettant de mettre en oeuvre le procédé indiqué ci-dessus.

Un tel kit de teinture comporte au moins deux compartiments, dont le premier renferme la composition (A) telle que définie ci-dessus et le second renferme la composition (B) comprenant un agent oxydant dans un milieu approprié pour la teinture.

D'autres objets de l'invention apparaîtront à la lecture de la description et des exemples qui suivent.

Les sels d'acide utilisés selon l'invention sont choisis de préférence parmi les chlorhydrates, les sulfates, les bromhydrates et les tartrates.

Le 3-méthyl para-aminophénol, le 2-méthyl para-aminophénol et le 2-hydroxyméthyl para-aminophénol ou leurs sels, sont présents à une concentration totale de 0,01% à 4% en poids par rapport au poids total de la composition tinctoriale, et de préférence de 0,1 à 2% en poids.

Le 2-méthyl 5-aminophénol ainsi que ses sels, représente de 0,005 à 5% en poids du poids total de la composition tinctoriale, et de préférence de 0,01 à 3,5% en poids.

Les paraphénylènediamines et/ou les bis-phénylalkylènediamines représentent de 0,01 à 8% en poids par rapport au poids total de la composition tinctoriale, et de préférence de 0,1 à 4% en poids.

L'ensemble précurseurs de colorants d'oxydation et coupleurs selon l'invention, représente de 0,1 à 10% en poids, et de préférence de 0,4 à 7% en poids, par rapport au poids total de la composition.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

La composition (A), qui renferme l'association des colorants telle que décrite ci-dessus, peut avoir un pH compris entre 3 et 10,5, qui peut être ajusté à la valeur choisie au moyen d'agents alcalinisants habituellement utilisés en teinture des fibres kératiniques, tels que l'ammoniaque, les carbonates alcalins, les alcanolamines, par exemple les mono-, di- et triéthanolamines ainsi que leurs dérivés, les hydroxydes de sodium ou de potassium, les composés de formule : 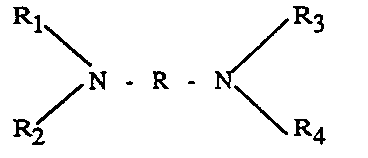 dans laquelle:
R est un reste propylène éventuellement substitué par un groupement hydroxyle ou un radical alkyle en C₁-C₄;
R₁, R₂, R₃ et R₄, simultanément ou indépendamment l'un de l'autre, représentent un atome d'hydrogène, un radical alkyle en C₁-C₄ ou hydroxyalkyle en C₁-C₄;
ou d'agents acidifiants classiques, tels que les acides minéraux ou organiques, par exemple les acides chlorhydrique, tartrique, citrique et phosphorique.

Le pH de la composition (B) renfermant l'agent oxydant tel que défini ci-dessus, est tel qu'après un mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que décrits ci-dessus.

La composition oxydante (B) est de préférence constituée par une solution d'eau oxygénée.

Selon un mode de réalisation préféré du procédé de teinture de l'invention, on mélange au moment de l'emploi la composition tinctoriale (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on les rince, les lave au shampooing, on les rince à nouveau et on les sèche.

Selon l'invention, les compositions tinctoriales peuvent contenir, en plus des colorants définis ci-dessus, d'autres coupleurs et/ou des colorants directs, notamment en vue de nuancer ou d'enrichir en reflets les colorations apportées par les précurseurs de colorants d'oxydation.

Ces coupleurs sont bien connus en eux-mêmes et sont choisis parmi l'α-naphtol; les dérivés indoliques; les coupleurs possédant un groupement méthylène actif, tels que les composés β-cétoniques; les pyrazolones; ainsi que leurs sels.

Les colorants directs sont de préférence des colorants azoïques, anthraquinoniques ou des dérivés nitrés de la série benzénique.

Les compositions tinctoriales conformes à l'invention contiennent également dans leur forme de réalisation préférée, des agents tensioactifs anioniques, cationiques, non-ioniques, amphotères ou leurs mélanges. Parmi ces agents tensio-actifs, on peut citer les alkylbenzènesulfonates, les alkylnaphtalènesulfonates, les sulfates, les éthersulfates et les sulfonates d'alcools gras, les alkylpolyglycosides, les sels d'ammonium quaternaire, tels que le bromure de triméthylcétylammonium, le bromure de cétylpyridinium, les éthanolamides d'acides gras éventuellement oxyéthylénés, les acides, alcools et amines polyoxyéthylénés, les alcools gras polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols comme le 2-butoxyéthanol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions conformes à l'invention, peuvent être choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite.

Ces agents épaississants sont présents de préférence dans des proportions comprises entre 0,1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tertiobutyl hydroquinone et l'acide homogentisique.

Ces agents antioxydants sont présents dans la composition dans des proportions comprises entre 0,05 et 1,5 % en poids par rapport au poids total de la composition.

Ces compositions peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. Ces compositions peuvent être conditionnées sous pression en flacons aérosols en présence d'un agent propulseur et former des mousses.

Les exemples qui suivent sont destinés à illustrer l'invention sans pour autant présenter un caractère limitatif.

### TEINTURE A pH BASIQUE

### EXEMPLES 1 à 4

On prépare la composition tinctoriale suivante :

| | |
|---|---|
| . Alcool oléique polyglycérolé à 2 moles de glycérol | 4,0 g |
| . Alcool oléique polyglycérolé à 4 moles de glycérol (78% de MA) | 5,7 g MA |
| . Acide oléique | 3,0 g |
| . Amine oléique oxyéthylénée à 2 moles d'oxyde d'éthylène, vendue sous la dénomination ETHOMEEN O12 par la Société AKZO | 7,0 g |
| . Laurylamino succinamate de diéthylaminopropyle, sel de sodium à 55% de MA | 3,0 g MA |
| . Alcool oléique | 5,0 g |
| . Diéthanolamide d'acide oléique | 12,0 g |
| . Propylèneglycol | 3,5 g |
| . Alcool éthylique | 7,0 g |
| . Dipropylèneglycol | 0,5 g |
| . Monométhyléther de propylèneglycol | 9,0 g |
| . Métabisulfite de sodium en solution aqueuse à 35% de MA | 0,46 g MA |
| . Acétate d'ammonium | 0,8 g |
| . Antioxydant, séquestrant qs | |
| . Parfum, conservateur qs | |
| . Ammoniaque à 20% de NH₃ | 10,0 g |
| . Colorants | x g. |
| . Eau déminéralisée qsp | 100 g |

Au moment de l'emploi, on mélange cette composition, poids pour poids, avec de l'eau oxygénée titrant 20 volumes (6% en poids), de pH 3.

On obtient un mélange de pH indiqué dans le tableau ci-après.

On applique ce mélange sur des cheveux gris à 90% de blancs, naturels ou permanentés, pendant 30 minutes. Après rinçage, lavage au shampooing, rinçage et séchage, les cheveux sont teints dans les nuances indiquées dans le tableau ci-après.

| Exemple | 1 | 2 | 3 | 4 |
|---|---|---|---|---|
| 3-méthyl p-aminophénol | 1 g | | 1 g | 1 g |
| 2-méthyl p-aminophénol | | 0,5 g | | |
| 2-hydroxyméthyl p-aminophénol | | 0,5 g | | |
| 2-méthyl 5-aminophénol | 1,5 g | 1,5 g | 1,5 g | 1,5 g |
| 2,6-diméthyl p-phénylènediamine, dichlorhydrate | 0,1 g | | 0,5 g | |
| 2-β-hydroxyéthyl p-phénylènediamine, dichlorhydrate | 0,1 g | | | 0,5 g |
| 4-amino N-(β-méthoxyéthyl)aniline, dichlorhydrate | 0,1 g | 0,15 g | | |
| N,N'-bis-(β-hydroxyéthyl)N,N'-bis-(4'-aminophényl)1,3-diamino 2-propanol, tétrachlorhydrate | 0,05 g | | | |
| N,N-bis-(β-hydroxyéthyl) p-phénylènediamine, sulfate | | 0,15 g | | |
| pH du mélange appliqué sur les cheveux | 9,7 | 9,8 | 9,6 | 9,6 |

| **NUANCE OBTENUE :** | | | | |
|---|---|---|---|---|
| . sur cheveux gris naturels à 90% blancs | Blond irisé cuivré | | | Blond irisé rouge cuivré |
| . sur cheveux gris permanentés à 90% blancs | | Blond foncé cuivré rouge | Rouge acajou | |

### TEINTURE A pH ACIDE

### EXEMPLES 5 et 6

On prépare la composition tinctoriale suivante :

| | |
|---|---|
| . Alcool oléique polyglycérolé à 2 moles de glycérol | 4,0 g |
| . Alcool oléique polyglycérolé à 4 moles de glycérol (78% de MA) | 5,7 g MA |
| . Acide oléique | 3,0 g |
| . Amine oléique oxyéthylénée à 2 moles d'oxyde d'éthylène, vendue sous la dénomination ETHOMEEN O12 par la Société AKZO | 7,0 g |
| . Laurylamino succinamate de diéthylaminopropyle, sel de sodium à 55% de MA | 3,0 g MA |
| . Alcool oléique | 5,0 g |
| . Diéthanolamide d'acide oléique | 12,0 g |
| . Propylèneglycol | 3,5 g |
| . Alcool éthylique | 7,0 g |
| . Dipropylèneglycol | 0,5 g |
| . Monométhyléther de propylèneglycol | 9,0 g |
| . Métabisulfite de sodium en solution aqueuse à 35% de MA | 0,46 g MA |
| . Acétate d'ammonium | 0,8 g |
| . Antioxydant, séquestrant qs | |
| . Parfum, conservateur qs | |
| . Monoéthanolamine | 0,8 g |
| . Colorants | x g |
| . Eau déminéralisée qsp | 100 g |

Le pH de la composition tinctoriale des exemples 5 et 6 avant mélange est de 8,8.

Au moment de l'emploi, on mélange la composition tinctoriale poids pour poids avec de l'eau oxygénée titrant 20 volumes (6% en poids) et dont le pH est ajusté à 1,4 par 1,7 g d'acide orthophosphorique pour 100 g d'eau oxygénée.

On obtient un mélange dont le pH est indiqué dans le tableau ci-après.

On applique ce mélange sur les cheveux pendant 30 minutes. Après rinçage, lavage au shampooing, rinçage et séchage, les cheveux sont teints dans les nuances indiquées dans le tableau ci-après.

| Exemple | 5 | 6 |
|---|---|---|
| 3-méthyl p-aminophénol | 1 g | 1 g |
| 2-méthyl 5-aminophénol | 1,5 g | 1,5 g |
| 2,6-diméthyl p-phénylènediamine, dichlorhydrate | 0,5 g | 0,5 g |
| 2-β-hydroxyéthyl p-phénylènediamine, dichlorhydrate | | 0,5 g |
| pH du mélange | 6,8 | 6,8 |

| **NUANCE OBTENUE :** | | |
|---|---|---|
| . sur cheveux gris naturels à 90% blancs | | Blond doré cendré irisé |
| . sur cheveux gris permanentés à 90% blancs | Blond foncé acajou cuivré | |

## Revendications

1. Composition tinctoriale pour fibres kératiniques, en particulier pour fibres kératiniques humaines telles que les cheveux, caractérisée par le fait qu'elle comprend, dans un milieu approprié pour la teinture :
- au moins un précurseur de colorant d'oxydation choisi parmi le 3-méthyl para-aminophénol, le 2-méthyl para-aminophénol et le 2-hydroxyméthyl para-aminophénol, et leurs sels d'addition avec un acide;
- au moins, à titre de coupleur, le 2-méthyl 5-aminophénol, ou l'un de ses sels d'addition avec un acide; et
- au moins un précurseur de colorant d'oxydation choisi parmi les paraphénylènediamines de formule (I) ci-après : 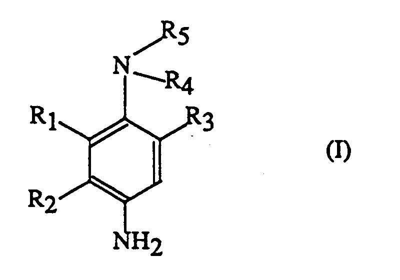 dans laquelle :
R₁, R₂, R₃, identiques ou différents, représentent un atome d'hydrogène ou d'halogène, un radical alkyle, un radical alcoxy, un radical carboxy, sulfo ou hydroxyalkyle en C₁-C₄ ;
R₄ et R₅, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino; ou bien R₄ et R₅ forment conjointement avec l'atome d'azote auquel ils sont liés, un hétérocycle pipéridino ou morpholino, sous réserve que R₁ ou R₃ représente un atome d'hydrogène lorsque R₄ et R₅ ne représentent pas un atome d'hydrogène, et sous réserve que lorsque R₁, R₂ et R₃ désignent un atome d'hydrogène, l'un des radicaux R₄ et R₅ soit différent d'hydrogène, et lorsque R₄ et R₅ désignent un atome d'hydrogène et que l'un des radicaux R₁, R₂ ou R₃ représente un radical méthyle, au moins l'un des deux autres soit différent d'hydrogène;
et les bis-phénylalkylènediamines de formule (II) suivante : 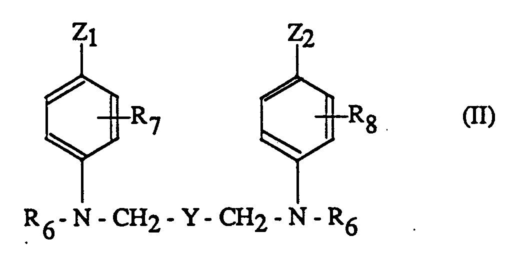 dans laquelle :
Z₁ et Z₂, identiques ou différents, représentent des groupements hydroxyle ou NHR₉, où R₉ désigne un atome d'hydrogène ou un radical alkyle ayant 1 à 4 atomes de carbone ;
R₇ et R₈, identiques ou différents, représentent soit des atomes d'hydrogène, soit des atomes d'halogène, soit encore des groupements alkyle ;
R₆ représente un atome d'hydrogène, un groupe alkyle, hydroxyalkyle ou aminoalkyle, dont le reste amino peut être substitué;
Y représente un radical pris dans le groupe constitué par les radicaux suivants :
-(CH₂)ₙ-, -(CH₂)ₘ-O-(CH₂)ₘ-,
-(CH₂)ₘ-CHOH-(CH₂)ₘ-, 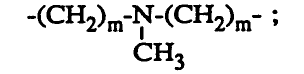 n étant un nombre entier compris entre 0 et 8 et m un nombre entier compris entre 0 et 4;
ainsi que leurs sels d'addition avec un acide.

2. Composition tinctoriale selon la revendication 1, caractérisée par le fait que le précurseur de colorant d'oxydation de type para est le 3-méthyl para-aminophénol ou l'un de ses sels d'addition avec un acide.

3. Composition tinctoriale selon la revendication 1 ou 2, caractérisée par le fait qu'elle contient une paraphénylènediamine de formule (I) choisie parmi la 2,6-diméthylparaphénylènediamine, la 2-β-hydroxyéthylparaphénylènediamine, la 2-hydroxyméthylparaphénylènediamine, la N-(β-hydroxypropyl)paraphénylènediamine, la 2-n-propylparaphénylènediamine, la 2-isopropylparaphénylènediamine, la N,N-di-(β-hydroxyéthyl)paraphénylènediamine, la 4-amino N-(β-méthoxyéthyl)aniline, et leurs sels d'addition avec un acide.

4. Composition tinctoriale selon l'une quelconque des revendications 1 à 3, caractérisée par le fait que la bisphénylalkylènediamine de formule (II) est le N,N'-bis-(β-hydroxyéthyl)N,N'-bis-(4'-aminophényl)1,3-diamino 2-propanol ou l'un de ses sels d'addition avec un acide.

5. Composition tinctoriale selon l'une quelconque des revendications 1 à 4, caractérisée par le fait que les sels d'addition avec un acide sont choisis parmi les chlorhydrates, les sulfates, les bromhydrates et les tartrates.

6. Composition tinctoriale selon l'une quelconque des revendications 1 à 5, caractérisée par le fait que le 3-méthyl paraaminophénol, le 2-méthyl para-aminophénol et le 2-hydroxyméthyl para-aminophénol ou leurs sels, sont présents dans une concentration totale de 0,01% à 4% en poids, et de préférence de 0,1 à 2% en poids par rapport au poids total de la composition.

7. Composition tinctoriale selon l'une quelconque des revendications 1 à 6, caractérisée par le fait que le 2-méthyl 5-aminophénol ou ses sels, est présent à une concentration de 0,005 à 5% en poids, et de préférence de 0,01 à 3,5% en poids par rapport au poids total de la composition.

8. Composition tinctoriale selon l'une quelconque des revendications 1 à 7, caractérisée par le fait que la paraphénylènediamine et/ou la bis-phénylalkylènediamine sont présentes à une concentration totale de 0,01 à 8% en poids, et de préférence de 0,1 à 4% en poids par rapport au poids total de la composition.

9. Composition tinctoriale selon l'une quelconque des revendications 1 à 8, caractérisée par le fait que les précurseurs de colorants d'oxydation et le coupleur sont présents dans une concentration totale de 0,1 à 10% en poids, et de préférence de 0,4 à 7% en poids par rapport au poids total de la composition.

10. Composition tinctoriale selon l'une quelconque des revendications 1 à 9, caractérisée par le fait qu'elle a un pH compris entre 3 et 10,5.

11. Composition tinctoriale selon l'une quelconque des revendications 1 à 10, caractérisée par le fait qu'elle contient d'autres coupleurs choisis parmi l'α-naphtol; les dérivés indoliques; les composés β-cétoniques; les pyrazolones; ainsi que leurs sels.

12. Composition tinctoriale selon l'une quelconque des revendications 1 à 11, caractérisée par le fait qu'elle contient en outre des colorants directs choisis parmi les colorants azoïques, anthraquinoniques et les dérivés nitrés de la série benzénique.

13. Composition tinctoriale selon l'une quelconque des revendications 1 à 12, caractérisée par le fait qu'elle contient en outre au moins un adjuvant choisi parmi les agents tensio-actifs anioniques, cationiques, non-ioniques, amphotères ou leurs mélanges en des proportions comprises entre 0,5 et 55% en poids, les solvants organiques en des proportions comprises entre 1 et 40% en poids, les agents épaississants en des proportions comprises entre 0,1 et 5% en poids, les agents antioxydants dans des proportions comprises entre 0,05 et 1,5% en poids, les proportions étant calculées par rapport au poids total de la composition, les agents de pénétration, les agents séquestrants, les parfums, les tampons, les agents dispersants, les agents de conditionnement, les agents filmogènes, les agents conservateurs et les agents opacifiants.

14. Agent de teinture des fibres kératiniques et en particulier des fibres kératiniques humaines telles que les cheveux, caractérisé par le fait qu'il comporte au moins deux composants : un composant (A) constitué par une composition de teinture selon l'une quelconque des revendications 1 à 13, et un composant (B) comprenant un agent oxydant dans un milieu approprié pour la teinture.

15. Agent de teinture selon la revendication 14, caractérisé par le fait que l'agent oxydant est choisi parmi le peroxyde d'hydrogène, le peroxyde d'urée, les bromates de métaux alcalins, les perborates et les persulfates.

16. Procédé de teinture des fibres kératiniques et en particulier des fibres kératiniques humaines telles que les cheveux, caractérisé par le fait qu'il consiste à appliquer sur les fibres une composition tinctoriale (A) selon l'une quelconque des revendications 1 à 13, et à révéler la couleur en milieu acide ou alcalin à l'aide d'un agent oxydant ajouté juste au moment de l'emploi à cette composition ou présent dans une composition (B) appliquée simultanément ou séquentiellement de façon séparée.

17. Procédé de teinture selon la revendication 16, caractérisé par le fait qu'on mélange au moment de l'emploi la composition tinctoriale selon l'une quelconque des revendications 1 à 13 avec une solution oxydante en une quantité suffisante pour développer une coloration, puis on applique le mélange obtenu sur les fibres, on laisse poser pendant 5 à 40 minutes, de préférence 15 à 30 minutes, puis on rince, on lave au shampooing, on rince à nouveau et on sèche.

18. Dispositif à plusieurs compartiments ou kit de teinture, caractérisé par le fait qu'il comporte au moins deux compartiments dont un premier compartiment renferme la composition (A) telle que définie dans l'une quelconque des revendications 1 à 13, et le second compartiment renferme la composition (B) comprenant un agent oxydant dans un milieu approprié pour la teinture.

## Claims

1. Dyeing composition for keratinous fibres, in particular for human keratinous fibres such as hair, characterized in that it comprises, in a suitable medium for dyeing:
- at least one oxidation dye precursor chosen from 3-methyl-para-aminophenol, 2-methyl-para-aminophenol and 2-hydroxymethyl-para-aminophenol, and their addition salts with an acid;
- as coupling agent, at least 2-methyl-5-aminophenol, or one of its addition salts with an acid; and
- at least one oxidation dye precursor chosen from the para-phenylenediamines of formula (I) below: 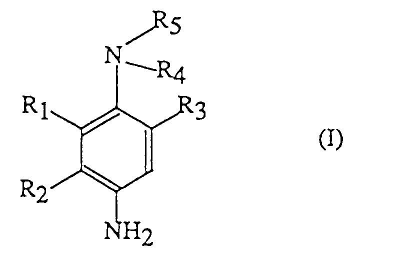 in which:
R₁, R₂ and R₃, which may be identical or different, represent a hydrogen or halogen atom, an alkyl radical, an alkoxy radical, a carboxyl or sulpho radical or a C₁-C₄ hydroxyalkyl radical;
R₄ and R₅, which may be identical or different, represent a hydrogen atom, an alkyl, hydroxyalkyl, alkoxyalkyl, carbamylalkyl, mesylaminoalkyl, acetylaminoalkyl, ureidoalkyl, carbalkoxyaminoalkyl, sulphoalkyl, piperidinoalkyl or morpholinoalkyl radical or a phenyl radical which is optionally para-substituted with an amino group; or alternatively R₄ and R₅ form, together with the nitrogen atom to which they are attached, a piperidino or morpholino heterocycle, with the proviso that R₁ or R₃ represents a hydrogen atom when R₄ and R₅ do not represent hydrogen atoms, and with the proviso that when R₁, R₂ and R₃ denote hydrogen atoms, one of the radicals R₄ and R₅ is other than hydrogen, and when R₄ and R₅ denote hydrogen atoms and one of the radicals R₁, R₂ or R₃ represents a methyl radical, at least one of the other two is other than hydrogen;
and the bis(phenylalkylenediamines) of formula (II) below: 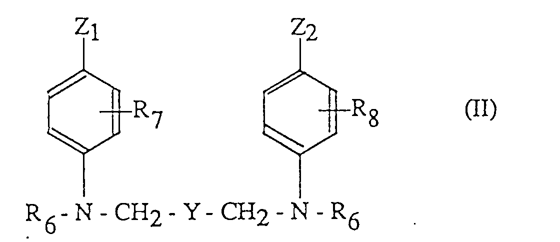 in which:
Z₁ and Z₂ , which may be identical or different, represent hydroxyl groups or groups NHR₉ , where R₉ denotes a hydrogen atom or an alkyl radical having 1 to 4 carbon atoms; R₇ and R₈, which may be identical or different, represent either hydrogen atoms or halogen atoms or alternatively alkyl groups;
R₆ represents a hydrogen atom or an alkyl, hydroxyalkyl or aminoalkyl group in which the amino residue may be substituted;
Y represents a radical taken from the group consisting of the following radicals:
-(CH₂)ₙ-, -(CH₂)ₘ-O-(CH₂)ₘ-,
-(CH₂)ₘ-CHOH-(CH₂)ₘ-, 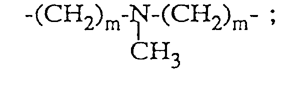 n being an integer between 0 and 8 and m an integer between 0 and 4;
as well as their addition salts with an acid.

2. Dyeing composition according to Claim 1, characterized in that the oxidation dye precursor of para type is 3-methyl-para-aminophenol or one of its addition salts with an acid.

3. Dyeing composition according to Claim 1 or 2, characterized in that it contains one para-phenylenediamine of formula (I) chosen from 2,6-dimethyl-para-phenylenediamine, 2-β-hydroxyethyl-para-phenylenediamine, 2-hydroxymethyl-para-phenylenediamine, N-(β-hydroxypropyl)para-phenylenediamine, 2-n-propyl-para-phenylenediamine, 2-isopropyl-para-phenylenediamine, N,N-di(β-hydroxyethyl)para-phenylenediamine, 4-amino-N-(β-methoxyethyl)aniline, and their addition salts with an acid.

4. Dyeing composition according to any one of Claims 1 to 3, characterized in that the bis(phenylalkylenediamine) of formula (II) is N,N'-bis(β-hydroxyethyl)N,N'-bis(4-aminophenyl)-1,3-diamino-2-propanol or one of its addition salts with an acid.

5. Dyeing composition according to any one of Claims 1 to 4, characterized in that the addition salts with an acid are chosen from hydrochlorides, sulphates, hydrobromides and tartrates.

6. Dyeing composition according to any one of Claims 1 to 5, characterized in that 3-methyl-para-aminophenol, 2-methyl-para-aminophenol and 2-hydroxymethyl-para-aminophenol or their salts are present in a total concentration of 0.01% to 4% by weight and preferably from 0.1 to 2% by weight relative to the total weight of the composition.

7. Dyeing composition according to any one of Claims 1 to 6, characterized in that 2-methyl-5-aminophenol or its salts is present at a concentration of 0.005 to 5% by weight and preferably from 0.01 to 3.5% by weight relative to the total weight of the composition.

8. Dyeing composition according to any one of Claims 1 to 7, characterized in that the para-phenylenediamine and/or the bis(phenylalkylenediamine) are present in a total concentration of 0.01 to 8% by weight and preferably from 0.01 to 4% by weight relative to the total weight of the composition.

9. Dyeing composition according to any one of Claims 1 to 8, characterized in that the oxidation dye precursors and the coupling agent are present in a total concentration of 0.1 to 10% by weight and preferably from 0.4 to 7% by weight relative to the total weight of the composition.

10. Dyeing composition according to any one of Claims 1 to 9, characterized in that it has a pH between 3 and 10.5.

11. Dyeing composition according to any one of Claims 1 to 10, characterized in that it contains other coupling agents chosen from α-naphthol; indole derivatives; β-keto compounds; pyrazolones; as well as their salts.

12. Dyeing composition according to any one of Claims 1 to 11, characterized in that it additionally contains direct dyes chosen from azo and anthraquinone dyes and nitro derivatives from the benzene series.

13. Dyeing composition according to any one of Claims 1 to 12, characterized in that it additionally contains at least one adjuvant chosen from anionic, cationic, nonionic and amphoteric surface-active agents or their mixtures in proportions between 0.5 and 55% by weight, organic solvents in proportions between 1 and 40% by weight, thickening agents in proportions between 0.1 and 5% by weight, and antioxidants in proportions between 0.05 and 1.5% by weight, the proportions being calculated relative to the total weight of the composition, penetration agents, sequestrating agents, perfumes, buffers, dispersing agents, conditioning agents, film-forming agents, preservatives and opacifying agents.

14. Dyeing agent for keratinous fibres and in particular for human keratinous fibres such as hair, characterized in that it contains at least two components: a component (A) consisting of a dyeing composition according to any one of Claims 1 to 13, and a component (B) comprising an oxidizing agent in a suitable medium for dyeing.

15. Dyeing agent according to Claim 14, characterized in that the oxidizing agent is chosen from hydrogen peroxide, urea peroxide, alkali metal bromates, perborates and persulphates.

16. Method of dyeing keratinous fibres and in particular human keratinous fibres such as hair, characterized in that it consists in applying to the fibres a dyeing composition (A) according to any one of Claims 1 to 13, and in developing the colour in an acidic or alkaline medium using an oxidizing agent which is added just at the moment of use to this composition or which is present in a composition (B) which is applied simultaneously or sequentially in a separate manner.

17. Method of dyeing according to Claim 16, characterized in that the dyeing composition according to any one of Claims 1 to 13 is mixed at the time of use with an oxidizing solution in a sufficient amount to develop a colouration, the mixture obtained is then applied to the fibres, it is left to stand for 5 to 40 minutes, preferably 15 to 30 minutes, and is then rinsed, washed with shampoo, rinsed again and dried.

18. Device containing several compartments or dyeing kit, characterized in that it contains at least two compartments, a first compartment of which contains the composition (A) as defined in any one of Claims 1 to 13, and the second compartment contains the composition (B) comprising an oxidizing agent in a suitable medium for dyeing.

## Patentansprüche

1. Färbezusammensetzung für keratinische, insbesondere menschliche keratinische Fasern wie die Haare,
dadurch **gekennzeichnet**, daß
sie, in einem zur Färbung geeigneten Milieu, umfasst:
- mindestens eine Oxidationsfarbstoff-Vorstufenverbindung, ausgewahlt aus 3-Methyl-p-aminophenol, 2-Methyl-p-aminophenol und aus 2-Hydroxymethyl-p-aminophenol und aus deren Additionssalzen mit einer Säure,
- mindestens, als Kuppler, 2-Methyl-5-aminophenol oder eines seiner Additionssalze mit einer Säure und
- mindestens eine Oxidationsfarbstoff-Vorstufenverbindung, ausgewählt aus p-Phenylendiaminen der folgenden Formel (I): 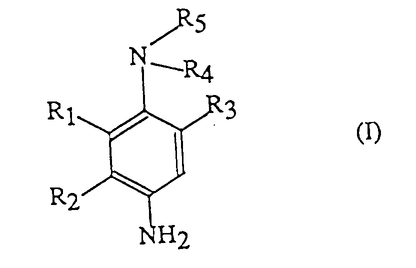 worin gilt:
R₁, R₂ und R₃ stellen, gleich oder verschieden, ein Wasserstoff- oder Halogenatom, einen Alkyl-, Alkoxy-, Carboxy-, Sulfo- oder einen C₁₋₄-Hydroxyalkylrest dar;
R₄ und R₅ stellen, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Sulfoalkyl-, Piperidinoalkyl-, Morpholinoalkyl- oder einen Phenylrest dar, der gegebenenfalls in para-Stellung mit einer Aminogruppierung substituiert ist, oder R₄ und R₅ bilden zusammen mit dem Stickstoffatom, an das sie gebunden sind, einen Piperidino- oder Morpholidino-Heterozyklus, mit der Maßgabe, daß R₁ oder R₃ ein Wasserstoffatom darstellen, wenn R₄ und R₅ kein Wasserstoffatom darstellen, und mit der Maßgabe, daß, wenn R₁, R₂ und R₃ ein Wasserstoffatom bedeuten, einer der Reste R₄ und R₅ von Wasserstoff verschieden ist, und wenn R₄ und R₅ ein Wasserstoffatom bedeuten und einer der Reste R₁, R₂ oder R₃ einen Methylrest darstellt, mindestens einer der beiden anderen Reste von Wasserstoff verschieden ist;
und aus Bisphenylalkylendiaminen der folgenden Formel (II): 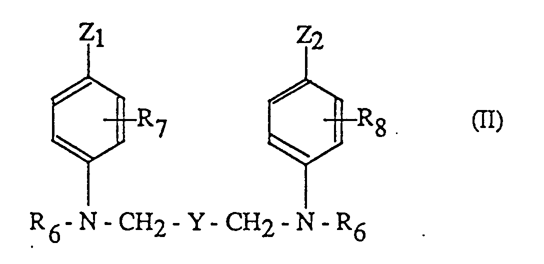 worin gilt:
Z₁ und Z₂ stellen, gleich oder verschieden, Hydroxyl- oder NHR₉-Gruppierungen dar, worin R₉ ein Wasserstoffatom oder einen Alkylrest mit 1 bis 4 Kohlenstoffatomen bedeutet;
R₇ und R₈ stellen, gleich oder verschieden, entweder Wasserstoffatome oder Halogenatome oder auch Alkylgruppen dar;
R₆ stellt ein Wasserstoffatom, eine Alkyl-, Hydroxyalkyl- oder eine Aminoalkylgruppe dar, deren Aminorest substituiert sein kann;
Y stellt einen Rest aus der Gruppe aus den folgenden Resten dar:
-(CH₂)ₙ-, -(CH₂)ₘ-O-(CH₂)ₘ-,
-(CH₂)ₘ-CHOH-(CH₂)ₘ-, 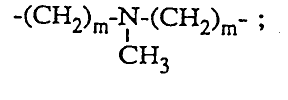 worin n eine ganze Zahl von 0 bis 8 und m eine ganze Zahl von 0 bis 4 sind,
sowie aus deren Additionssalzen mit einer Säure.

2. Färbezusammensetzung nach Anspruch 1,
dadurch **gekennzeichnet**, daß
die Oxidationsfarbstoff-Vorstufenverbindung vom para-Typ 3-Methyl-p-aminophenol oder eines seier Additionssalze mit einer Säure ist.

3. Färbezusammensetzung gemäß Anspruch 1 oder 2,
dadurch **gekennzeichnet**, daß
sie ein p-Phenylendiamin der Formel (I) enthält, ausgewählt aus 2,6-Dimethyl-p-phenylendiamin, 2-β-Hydroxyethyl-p-phenylendiamin, 2-Hydroxymethyl-p-phenylendiamin, 2-n-Propyl-p-phenylendiamin, 2-Isopropyl-p-phenylendiamin, N-(β-Hydroxypropyl)-p-phenylendiamin, N,N-Di(β-hydroxy-ethyl)-p-phenylendiamin, 4-Amino-N-(β-methoxyethyl)anilin sowie aus deren Additionssalzen mit einer Säure.

4. Färbezusammensetzung gemäß einem der Ansprüche 1 bis 3,
dadurch **gekennzeichnet**, daß
das Bisphenylalkylendiamin der Formel (II) N,N'-Bis(β-hydroxyethyl)-N,N'-bis(4'-aminophenyl)-1,3-diaminopropan-2-ol oder eines seiner Additionssalze mit einer Säure ist.

5. Färbezusammensetzung gemäß einem der Ansprüche 1 bis 4,
dadurch **gekennzeichnet**, daß
die Additionssalze mit einer Säure aus Hydrochloriden, Sulfaten, Hydrobromiden und aus Tartraten ausgewählt sind.

6. Färbezusammensetzung nach einem der Ansprüche 1 bis 5,
dadurch **gekennzeichnet**, daß
das 3-Methyl-p-aminophenol, das 2-Methyl-p-aminophenol und das 2-Hydroxymethyl-p-aminophenol oder deren Salze in einer Gesamtkonzentration von 0,01 bis 4 und vorzugsweise von 0,1 bis 2 Gew.% vorhanden sind, bezogen auf das Gesamtgewicht der Zusammensetzung.

7. Färbezusammensetzung gemäß einem der Ansprüche 1 bis 6,
dadurch **gekennzeichnet**, daß
das 2-Methyl-5-aminophenol oder seine Salze in einer Konzentration von 0,005 bis 5 und vorzugsweise von 0,01 bis 3,5 Gew.% vorhanden ist, bezogen auf das Gesamtgewicht der Zusammensetzung.

8. Färbezusammensetzung gemäß einem der Ansprüche 1 bis 7,
dadurch **gekennzeichnet**, daß
das p-Phenylendiamin und/oder das Bisphenylalkylendiamin in einer Gesamtkonzentration von 0,01 bis 8 und vorzugsweise von 0,1 bis 4 Gew.% vorhanden sind, bezogen auf das Gesamtgewicht der Zusammensetzung.

9. Färbezusammensetzung gemäß einem der Ansprüche 1 bis 8,
dadurch **gekennzeichnet**, daß
die Oxidationsfarbstoff-Vorstufenverbindungen und der Kuppler in einer Gesamtkonzentration von 0,1 bis 10 und vorzugsweise von 0,4 bis 7 Gew.% vorhanden sind, bezogen auf das Gesamtgewicht der Zusammensetzung.

10. Färbezusammensetzung gemäß einem der Ansprüche 1 bis 9,
dadurch **gekennzeichnet**, daß
sie einen pH-Wert von 3 bis 10,5 aufweist.

11. Färbezusammensetzung gemäß einem der Ansprüche 1 bis 10,
dadurch **gekennzeichnet**, daß
sie weitere Kuppler enthält, ausgewählt aus α-Naphthol, Indolderivaten, β-Ketoverbindungen, Pyrazolonen sowie aus deren Salzen.

12. Färbezusammensetzung gemäß einem der Ansprüche 1 bis 11,
dadurch **gekennzeichnet**, daß
sie ausserdem Direktfarbstoffe enthält, ausgewählt aus Azo-, Anthrachinon-Farbstoffen und aus nitrierten Derivaten der Benzolreihe.

13. Färbezusammensetzung gemäß einem der Ansprüche 1 bis 12,
dadurch **gekennzeichnet**, daß
sie ausserdem mindestens einen Hilfsstoff, ausgewählt aus anionischen, kationsichen, nicht-ionischen und amphoteren oberflächenaktiven Mitteln oder aus deren Mischungen, in Mengenanteilen von 0,5 bis 55 Gew.%, aus organischen Lösungsmitteln in Mengenanteilen von 1 bis 40 Gew.%, aus Verdickungsmitteln in Mengenanteilen von 0,1 bis 5 Gew.%, aus Antioxidantien in Mengenanteilen von 0,05 bis 1,5 Gew.%, wobei die jeweiligen Mengenanteile unter Bezug auf das Gesamtgewicht der Zusammensetzung berechnet sind, aus Eindringmitteln, Sequestriermitteln, Parfüm-Produkten, Puffermitteln, Dispergiermitteln, Konditioniermitteln, filmbildenden Mitteln, Konservierungsmitteln und aus opak machenden Mitteln, enthält.

14. Mittel zur Färbung keratinischer und insbesondere menschlicher keratinischer Fasern wie der Haare,
dadurch **gekennzeichnet**, daß
es mindestens zwei Bestandteile umfaßt: einen Bestandteil (A), der aus einer Färbezusammensetzung gemäß jedem der Ansprüche 1 bis 13 zusammengesetzt ist, und einen Bestandteil (B), der ein oxidierendes Mittel in einem zur Färbung geeigneten Milieu umfaßt.

15. Mittel zur Färbung gemäß Anspruch 14,
dadurch **gekennzeichnet**, daß
das oxidierende Mittel aus Wasserstoffperoxid, Harnstoffperoxid, Alkalibromaten, Perboraten und aus Persulfaten ausgewählt ist.

16. Verfahren zur Färbung keratinischer und insbesondere menschlicher keratinischer Fasern wie der Haare,
dadurch **gekennzeichnet**, daß
man auf die Fasern eine Färbezusammensetzung gemäß jedem der Ansprüche 1 bis 13 aufbringt und die Farbe in saurem oder alkalischem Milieu mit einem oxidierenden Mittel entwickelt, das genau zum Zeitpunkt der Anwendung dieser Zusammensetzung zugefügt wird oder in einer Zusammensetzung (B) vorhanden ist, die gleichzeitig oder nacheinander aufgebracht wird.

17. Verfahren zur Färbung gemäß Anspruch 16,
dadurch **gekennzeichnet**, daß
man zum Zeitpunkt der Anwendung die Färbezusammensetzung gemäß jedem der Ansprüche 1 bis 13 mit einer oxidierenden Lösung in einer zur Entwicklung einer Färbung ausreichenden Menge vermischt, dann die erhaltene Mischung auf die Fasern aufbringt, das Ganze 5 bis 40 und vorzugsweise 15 bis 30 Minuten lang verweilen läßt und man dann spült, unter Schamponieren wäscht, erneut spült und trocknet.

18. Vorrichtung aus mehreren Teilen oder Kit zur Färbung,
dadurch **gekennzeichnet**, daß
sie mindestens zwei Teile umfassen, deren erster Teil die in jedem der Ansprüche 1 bis 13 definierte Zusammensetzung (A) und deren zweiter Teil die Zusammensetzung (B) umfassen, die ein oxidierendes Mittel in einem zur Färbung geeigneten Milieu enthält.
